# EUROPÄISCHE PATENTSCHRIFT

(11) **EP 3 224 614 B1**
(45) Veröffentlichungstag und Bekanntmachung des Hinweises auf die Patenterteilung: **05.09.2018**
(21) Anmeldenummer: 15778931.4
(22) Anmeldetag: 09.10.2015
(51) Int. Cl.: G01N 33/20

(54) **MESS- UND PROBEENTNAHMELANZE UND VERFAHREN**
MEASURING AND SAMPLING LANCE AND METHOD
LANCE DE MESURE ET DE PRÉLÈVEMENT D'ÉCHANTILLONS ET PROCÉDÉ

(30) Priorität: 27.11.2014 EP 14195122
(43) Veröffentlichungstag der Anmeldung: 04.10.2017
(73) Patentinhaber: Primetals Technologies Austria GmbH, 4031 Linz (AT)
(72) Erfinder: ROHRHOFER, Andreas, 4020 Linz (AT); LEHOFER, Martin, Plainsboro, NJ 08536 (US); PROFELT, Franz, 5723 Uttendorf (AT); KEUSCH, Thomas, 4020 Linz (AT); HARTL, Franz, 4720 Kallham (AT); FISCHER, Paul, 4040 Linz (AT); WEINZINGER, Michael, 4501 Neuhofen a. d. Krems (AT)
(74) Vertreter: Metals@Linz
(86) Internationale Anmeldenummer: PCT/EP2015/073405
(87) Internationale Veröffentlichungsnummer: WO 2016/083006

(56) Entgegenhaltungen:
- EP-A1- 1 617 194
- EP-A1- 1 813 919
- EP-A2- 1 614 758
- WO-A1-03/060432
- US-A1- 2012 278 003

## Beschreibung

Die Erfindung betrifft eine Mess- und Probeentnahmelanze und ein Verfahren zum Messen metallurgischer Kenngrößen und zum Ziehen einer Probe flüssiger Schmelze, wobei die Mess- und Probeentnahmelanze ein Kontaktstück und eine lösbare am Kontaktstück befestigbare Tauchsonde umfasst, wobei die Tauchsonde eintauchbar ist und zumindest einen Sensor zur Ausgabe eines für die Schmelze charakteristischen Sensorsignals aufweist.

Bei der Erzeugung und Verarbeitung von Flüssigmetallschmelzen, wie z.B. bei BOF, AOD, L TS, RH und CC müssen chemische Proben entnommen werden und physikalische Parameter wie z.B. die Temperatur bestimmt werden. Die gemessenen Werte sind für die weitere Bearbeitung immens wichtig, da die weiteren Bearbeitungsschritte sowie die Qualität des Stahls davon abhängig sind.

Zur Überwachung des Produktionsprozesses bei der Erzeugung von Flüssigmetallschmelzen, wie zum Beispiel in einem Stahlwerk, werden regelmäßig Messungen durchgeführt, z. B. der Temperatur oder des Sauerstoff- und Kohlenstoffgehaltes der flüssigen Schmelze. Diese Messungen werden je Schmelze mindestens einmal durchgeführt. Die Ergebnisse der Messungen fließen direkt in die Steuereinheit des metallurgischen Prozesses ein. Weiterhin werden die Ergebnisse in Qualitätsaufzeichnungen festgehalten.

Eine Messung läuft dabei beispielsweise wie folgt ab: eine Tauchsonde mit integrierter Sensorik wird mittels eines Kontaktstücks, welches eine Schnittstelle zur mechanischen und elektrischen Kopplung darstellt, auf einer Lanze, aufgesteckt. Die Leitungen der Sensoren werden über Steckkontakte durch den Manipulator bis zum Mess- bzw. Analysegerät geführt.

Ein Manipulator bewegt entweder automatisch die Tauchsonde in die Flüssigmetallschmelze, worauf die Messungen durchgeführt werden oder die Lanze mit der Tauchsonde wird durch einen Bediener manuell in die Flüssigmetallschmelze eingeführt. Ein Kabel führt Signale aus der Tauchsonde über das Kontaktstück und der Lanze zu einem Auswertegerät.

Die Messung mit kabelgebundenen Lanzen bringt folgende Probleme mit sich: durch Schlacken bzw. Stahlspritzer, Schrottteile und dergleichen ist das Kabel auch hohen Beanspruchungen ausgesetzt und wird häufig beschädigt. Je nach Grad der Beschädigung ist das Kabel sofort defekt oder - weitaus gefährlicher - es werden Fehlmessungen durchgeführt. Die zur Verwendung kommende Ausgleichsleitung ist jedoch sehr kostenintensiv.

Stahlwerke werden für gewöhnlich mit zwei oder drei Konvertern ausgeführt, die jeweils mit einer Lanze ausgestattet werden. Ausfälle des Messsystems sind gewöhnlich durch defekte Kabel bedingt, d.h. bis zum Anschluss des Ersatzkabels muss die Lanze des anderen Konverters benutzt werden. Der mehrmalige Wechsel des Kabels benötigt mehrere Minuten und wird durch die Steifigkeit des Kabels behindert. In Abhängigkeit vom eingesetzten Bedienpersonal variiert die Messqualität. Die Genauigkeit der Messung ist z.B. von der Eintauchtiefe und Eintauchgeschwindigkeit der Sonde in die Flüssigmetallschmelze abhängig. Ein zu langsames Eintauchen führt zu einem frühzeitigen Verbrennen der Sonde und zu einer Falschmessung. Wird die Sonde nicht tief genug eingetaucht, wird die Temperatur der Schlacke gemessen, nicht jedoch die Temperatur des Flüssigmetallschmelze. Das Eintauchen ist kraftaufwändiger durch den höheren Auftrieb der Flüssigmetallschmelze im Vergleich zu Wasser. Für die Durchführung der Messung ist also eine gewisse Erfahrung notwendig. Durch die Verarbeitung der Werte am Auswertegerät müssen die Signale kontinuierlich versendet werden, da die Elektronik der Lanze nicht entscheiden kann, wann eine Messung gestartet hat. Dies führt zu einer relativ kurzen Betriebsdauer der Lanze, da häufig der Akku nachgeladen werden muss. Die Beurteilung, ob eine Messung erfolgreich war oder nicht, erfolgt am Auswertegerät im Leitstand. Dadurch entsteht das Problem, wie der Bediener z.B. am Konverter davon informiert wird, ob eine Messung erfolgreich war oder nicht.

Aus der US 2012/278003 A1, EP 1 614 758 A2, EP 1 617 194 A1 WO 03/060432 A1 und EP 1 813 919 A1 sind verschiedene Ausgestaltungen von Messlanzen zum Messen metallurgischer Kenngrößen bekannt. Die aus der EP 1 614 758 A2 und der EP 1 813 919 A1 bekannten Mess- und Probenentnahmelanzen ermöglichen darüber hinaus auch noch das Ziehen einer Probe flüssiger Schmelze. Eine erste Aufgabe der Erfindung ist daher die Angabe einer Mess- und Probeentnahmelanze zum Messen metallurgischer Kenngrößen und zum Ziehen einer Probe flüssiger Schmelze, welche die oben genannten Probleme löst. Eine weitere, zweite Aufgabe ist die Angabe eines Verfahrens, welches sich insbesondere mit der erfindungsgemäßen Mess- und Probeentnahmelanze durchführen lässt.

Die erste Aufgabe wird daher durch die Angabe einer Mess- und Probeentnahmelanze zum Messen metallurgischer Kenngrößen und zum Ziehen einer Probe flüssiger Schmelze mit einem Gehäuse mit einer Elektronikeinheit gelöst, wobei die Mess- und Probeentnahmelanze ein Kontaktstück und eine lösbar am Kontaktstück befestigbare Tauchsonde umfasst. Dabei ist die Tauchsonde eintauchbar und weist zumindest einen Sensor zur Ausgabe eines für die Schmelze charakteristischen Sensorsignals auf. Die Mess- und Probenentnahmelanze weist das Gehäuse mit der Elektronikeinheit auf, insbesondere an der sensorabgewandten Seite der Mess- und Probeentnahmelanze, wobei der Sensor über das Kontaktstück mit dem Gehäuse durch eine elektrische Verbindung verbunden ist, so dass das Sensorsignal über die elektrische Verbindung zu der Elektronik einheit führbar ist, und wobei die Elektronikeinheit zumindest eine analoge Verarbeitungseinheit zum anlogen Vorverarbeiten des Sensorsignals umfasst und wobei die Elektronikeinheit einen Beschleunigungssensor zum Aufzeichnen des Eintauchvorgangs umfasst und die Elektronikeinheit dazu eingerichtet ist, über den Beschleunigungssensor den Eintauchvorgang aufzuzeichen.

Die auf das Verfahren bezogene Aufgabe wird gelöst durch die Angabe eines Verfahrens zum Messen metallurgischer Kenngrößen und zum Ziehen einer Probe flüssiger Schmelze mit einer Mess- und Probeentnahmelanze, die ein Gehäuse mit einer Elektronikeinheit aufweist, in welcher eine analoge Verarbeitungseinheit angeordnet ist, mit einem Kontaktstück und einer lösbar am Kontaktstück befestigbaren Tauchsonde, welche zumindest einen Sensor zur Ausgabe eines für die Schmelze charakteristischen Sensorsignals aufweist, welcher über das Kontaktstück (3,30) mit dem Gehäuse durch eine elektrische Verbindung verbunden ist, so dass das Sensorsignal über die elektrische Verbindung zu der Elektronikeinheit (6,60) geführt wird, wobei die Tauchsonde in die flüssige Schmelze zumindest teilweise eingetaucht wird, wobei der Sensor das für die Schmelze charakteristische Signal ausgibt und wobei durch eine in der Elektronikeinheit angeordnete, analoge Verarbeitungseinheit das Sensorsignal zumindest analog vorverarbeiten wird. Der Eintauchvorgang wird durch einen Beschleunigungssensor in der Elektronikeinheit aufgezeichnet.

In der Elektronikeinheit wird eine elektrische Verbindung, welche für gewöhnlich als Kabel ausgeführt ist, und wobei die elektrische Verbindung nachfolgend parallel zum besseren Verständnis auch so bezeichnet wird, vom Sensor über das Kontaktstück hineingeführt. In der Elektronikeinheit kann eine analoge Vorverarbeitung, insbesondere eine Verstärkung und eine Filterung, durchgeführt werden. Vorteilhafterweise umfasst die Verarbeitungseinheit einen A/D-Wandler zum Digitalisieren des Sensorsignals und eine Analyseeinheit zum Bestimmen zumindest eines vorab bestimmten Parameters aus dem digitalisierten Signal. Die Analyseeinheit verarbeitet das digitalisierte Signal und berechnet die physikalischen Parameter wie z.B. Temperatur, Sauerstoffgehalt und Kohlenstoffgehalt. Zusätzlich kann die Analyseeinheit weitere Sensoren bei der Ermittlung der Parameter berücksichtigen. Idealerweise ergibt sich somit ein Wegfall des Auswertegerätes, da alle Berechnungen bereits in der Elektronikeinheit stattfinden. Da die Elektronikeinheit alle Berechnungen vor Ort an der Lanze durchführt, führen Übertragungsfehler zu keinem Messabbruch und somit totalem Verlust der Messung. Messdaten können vorteilhafterweise bis nach einer erfolgreichen Datenübertragung zum Automatisierungssystem gespeichert werden. Zudem umfasst die Elektronikeinheit einen Beschleunigungssensor zum Aufzeichnen des Eintauchvorgangs. In einer bevorzugten Ausführung erstellt die Elektronikeinheit dann aus dem aufgezeichneten Eintauchvorgang ein Wegprofil des Eintauchvorgangs. Anhand dieses Wegprofils ist der Eintauchvorgang als gültig oder ungültig einstufbar, d.h., dass über den Beschleunigungssensor der Eintauchvorgang aufgezeichnet wird. Die Analyseeinheit ermittelt aus dem aufgezeichneten Eintauchvorgang ein Wegprofil des Eintauchvorgangs der Tauchsonde. Anhand solch eines Wegprofils ermittelt die Analyseeinheit, ob der Eintauchvorgang korrekt durchgeführt wurde. War der Messvorgang beispielsweise gültig, berechnet die Analyseeinheit einen Messwert für beispielsweise die Temperatur und/oder den Sauerstoffgehalt und/oder den Kohlenstoffgehalt der Flüssigmetallschmelze. Sind die Messspannungswerte berechnet, werden diese gemeinsam über eine Schnittstelle an ein Automatisierungssystem gesendet. Ist der Messvorgang, unabhängig davon, ob er gültig oder nicht gültig war, abgeschlossen, wird dem Bedienpersonal akustisch, optisch oder über Vibration mitgeteilt, dass er die Lanze aus der Schmelze ziehen muss, um diese vor Beschädigungen zu bewahren. Durch eine Auswertung des Wegprofils kann daher durch Abschätzung der Messqualität eine Detektion von ungenau ausgeführten Messvorgängen ermittelt werden. Zudem ist es so möglich, die Eintauchtiefe zu erfassen.

In den Unteransprüchen sind weitere vorteilhafte Maßnahmen aufgelistet, die beliebig miteinander kombiniert werden können, um weitere Vorteile zu erzielen.

In bevorzugter Ausgestaltung ist am Gehäuse eine Sendeeinheit mit Antenne vorgesehen, zum Senden zumindest des durch die analoge Verarbeitungseinheit analog vorverarbeiteten Sensorsignals an ein Automatisierungssystem, d.h. dass jeder Messvorgang über eine Sendeeinheit mit Antenne an ein Automatisierungssystem versendet wird. Die Übertragung der Daten kann z.B. über WirelessHART oder Industrial WLAN oder einem geeigneten RFID System erfolgen. Eine Ausführung ist auch mit jeder anderen geeigneten Übertragungstechnik, wie beispielsweise Bluetooth, denkbar. Im Automatisierungssystem werden die Daten weiter verarbeitet und z.B. anderen Automatisierungssystemen und/oder Prozessmodellen zugeführt und/oder einem Bediener angezeigt. Dadurch entstehen keine Ausfälle durch beschädigte Kabelverbindungen, wenn die Lanze kabellos ausgeführt wird.

Bevorzugt umfasst die Elektronikeinheit einen Speicher, insbesondere einen Ringspeicher. In einer vorzugsweisen Ausführung der Erfindung werden die aufgezeichneten Daten der Sensoren der Tauchsonde und der Analyseeinheit in einem Speicher verspeichert, der z.B. als Ringspeicher ausgeführt sein kann. Je nach Ausführung der Datenübertragung werden die aufgezeichneten Daten nach Abschluss der Übertragung des Messvorganges über dasselbe Medium versandt (z.B. WLAN). Wird der Messvorgang z.B. über WirelessHART versendet, können die Messdaten nachträglich über eine separate WLAN-Schnittstelle versandt werden. Genauso ist es vorstellbar, dass die Messdaten nicht aktiv versandt werden, sondern passiv von einem IT-System aus dem Speicher abgerufen werden. Beim passiven Abrufen fungiert die Lanze als Server und nicht als Client.

Bevorzugt sind auf der Mess- und Probeentnahmelanze und/oder an dem Gehäuse Zustandsindikatoren, welche bevorzugt visuell sind, d.h. dass der Zustand, in welchem sich die Mess- und Probeentnahmelanze befindet, durch einen Farbcode anzeigbar ist. Hier werden auf der Lanze und/oder auf der Elektronikeinheit visuelle Zustandsindikatoren angebracht. Die Zustandsindikatoren können z.B. als Multi-Color LED ausgeführt werden. Je nach Status der Messung, z.B. Messung läuft oder Messung abgeschlossen, können die LED in verschiedenen Farben leuchten.

In einem besonders bevorzugten Ausführungsbeispiel ist nach einem vorab festgelegten Zeitraum nach Entfernen der Tauchsonde die Elektronikeinheit in einen Stromsparmodus versetzbar. Bei Feststellen der Benutzung der Mess- und Probeentnahmelanze durch die Elektronikeinheit ist der Stromsparmodus aufhebbar, d.h. die Elektronik fährt beispielsweise nach einem definierten Zeitraum (z.B. 1 min) nach Entfernen der Tauchsonde in einen Stromsparmodus bei dem Sensoren bzw. Antennen abgeschaltet werden, oder in einen Low-Power-Modus versetzt werden. Der Stromsparmodus kann auch aktiviert werden, wenn über einen definierten Zeitraum kaum oder keine Bewegungen von den Beschleunigungssensoren detektiert werden. Die Elektronik prüft im Stromsparmodus von Zeit zu Zeit, ob eine Tauchsonde angesteckt wurde, z.B. durch eine Widerstandsmessung an dem Kontaktstück, oder fragt die Beschleunigungsdaten ab. Wird festgestellt, dass die Lanze wieder benutzt wird, werden alle Bauteile aus dem Stromsparmodus geweckt und eine Netzwerkverbindung hergestellt.

Vorzugweise sind entweder Hilfsmittel zur eindeutigen drahtlosen Identifizierung der Mess- und Probeentnahmelanze auf die Mess- und Probeentnahmelanze aufgebracht und/oder durch die Mess- und Probeentnahmelanze ist selbst eine Ortung vornehmbar. Damit wird eine automatische Zuordnung der Lanzen zu einer Messposition ermöglicht. So werden entweder auf der Lanze eindeutige Identifizierungen angebracht werden (z.B. Barcodes, QR-Codes, RFIDTags), die drahtlos ausgelesen werden können oder die Lanze kann selbst eine Ortung vornehmen.

Bevorzugt umfasst die Elektronikeinheit als einen ersten Schaltkreis einen Wandler zum Digitalisieren des Sensorsignals und eine Analyseeinheit und einen zusätzlichen Schaltkreis zur Durchführung einer Selbstdiagnose der Mess- und Probeentnahmelanze, wodurch der zusätzliche Schaltkreis durch einen Schalter in den ersten Schaltkreis zu- und weggeschaltbar ist und in dem zusätzlichen Schaltkreis ein Spannungsgenerator, insbesondere ein Digital-Analog-Umsetzer, vorgesehen ist.

Üblicherweise wird die Lanze von einem Bediener oder auch Manipulator in die Flüssigmetallschmelze getaucht. Bei diesem Eintauchvorgang wird die Tauchsonde zerstört (verbrannt). Wird die Lanze nach Beendigung des Messvorganges nicht schnell genug aus der Flüssigmetallschmelze gezogen, können die Kontakte des Kontaktstücks stark verschmutzt oder sogar zerstört werden. Wird das Equipment nicht in regelmäßigen Zeitintervallen auf korrekte Funktion hin überprüft, liefert die Lanze falsche Messergebnisse. Liegt eine Abweichung vor, muss das System gereinigt, oder das Kontaktstück ausgetauscht werden. Eine Neu-Kalibrierung der Elektronik ist nicht möglich. Diese unbedingt notwendigen Funktionsüberprüfungen werden aber nicht oder nur unzuverlässig oft durchgeführt. Daraus resultieren unweigerlich unerkannte Falschmessungen. Vorteilhafterweise wird nun die Analyse-Elektronik in die Mess- und Probeentnahmelanze vollständig integriert. Mithilfe dieser Analyse-Elektronik, kann sich die Mess- und Probeentnahmelanze selbst diagnostizieren und auch kalibrieren. In der Elektronikeinheit der Elektronik wird bevorzugt ein Digital/Analog-Umsetzer (D/A-Umsetzer) hinzugefügt. Dieser Digital/Analog-Umsetzer ist ein hochgenauer Spannungsgenerator, mit welchem die in der Tauchsonde enthaltenen Sensoren, welche Spannungen liefern, simuliert werden können.

Bevorzugt ist ein Kurzschlussadapter vorgesehen, in welcher die Tauchsonde einsteckbar ist. Bei Zuschalten des zusätzlichen Schaltkreises führt die Analyseeinheit dem Spannungsgenerator zumindest einen vorgegebenen Spannungssollwert zu, welcher durch den Spannungsgenerator in den zusätzlichen Schaltkreis generiert wird und wobei der Spannungssollwert durch eine elektrische Verbindung über das Kontaktstück an den Kurzschlussadapter führbar ist und anschließend über eine elektrische Verbindung an einen Wandler als Messspannungswert zurückführbar und dort digitalisierbar ist. Anschließend ist der digitalisierte Messspannungswert der Analyseeinheit zuführbar.

In diesem Modus wird in den Messkreis ein Spannungsgenerator eingekoppelt. Dadurch wird zusätzlich eine hochpräzise Spannungsquelle in den Messkreis geschalten. In dieser Betriebsart/diesem Betriebsmodus befindet sich der Schalter in Y-Stellung.

Der Spannungsgenerator kann dabei vorzugsweise als Digital/Analog-Umsetzer ausgeführt werden. Die Analyseeinheit gibt dem Digital/Analog-Umsetzer Spannungssollwerte für die Testspannung vor. Mit diesem Spannungsgenerator werden die Sensorspannungen einer Tauchsonde simuliert. Diese werden in weiterer Folge durch die Y-Stellung des Schalters in Serie als Spannungen in den Messkreis eingekoppelt. Über die elektrische Verbindung werden die Spannungssollwerte an das Kontaktstück weitergegeben, wo sie über den Kurzschlussadapter direkt als Messspannungswerte wieder zurück an den A/D-Wandler der Lanze zurückgeführt und somit gemessen werden können. Der A/D-Wandler misst nun die Spannung, welche vom D/A-Umsetzer erzeugt wurde. Treten irgendwo im Messkreis Spannungsverluste in Form von erhöhten Übergangswiderständen oder durch Leitungsbruch auf, kann dies durch den A/D-Wandler erkannt werden. Fehler im Messkreis können von der Analyseeinheit somit erkannt und es kann darauf reagiert werden.

Bevorzugt ist der Wandler ein A/D-Wandler zum Digitalisieren des Sensorsignals, wobei der A/D-Wandler bei Wegschalten des zusätzlichen Schaltkreises das digitalisierte Sensorsignal an die Analyseeinheit leitet, zum Bestimmen zumindest eines vorab bestimmten Parameters aus dem digitalisierten Signal. In diesem Betriebsmodus werden die Tauchsonden auf die Lanze aufgesteckt. Die Elektronik erfasst die Sensorwerte, gibt sie an die Analyseeinheit weiter, welche die Analyseeinheit aufbereitet und einem Leitsystem zur Verfügung stellt. In dieser Betriebsart funktioniert die Lanze weiter wie eine oben beschriebene Lanze. Der Schalter ist in X-Stellung, wodurch die von der Tauchsonde generierten Sensorspannungssignale über das Kontaktstück und die elektrische Verbindung direkt an den Analog/Digital-Wandler der Messelektronik geleitet werden.

Der Analog/Digital-Wandler wandelt die Spannungssignale, beispielsweise von einem in der Tauchsonde befindlichen Thermoelement, in einen digitalen Messwert und gibt diesen an die Analyseeinheit weiter.

Bevorzugt sind mehrere verschiedene Spannungssollwerte vorgesehen, welche bei Zuschalten des zusätzlichen Schaltkreises durch die Analyseeinheit dem Spannungsgenerator zuführbar sind. Dies ist vorteilhaft, da die Tauchsonde mehrere Tauchsondensensoren aufweist, welche durch die mehreren, verschiedenen Spannungssollwerte diagnostizierbar sind. Der sequentielle Test von mehreren verschiedenen Spannungen für jeden einzelnen in der Tauchsonde eingebauten Sensor, mit welchen hintereinander der Messkreis überprüft wird, ist daher von Vorteil.

Bevorzugt ist durch die Analyseeinheit der zusätzliche Schaltkreis durch den Schalter automatisiert in den ersten Schaltkreis zu- und wegschaltbar, d.h. die Analyseeinheit bringt automatisch den Schalter in die notwendige X-Stellung oder Y-Stellung. In diesem Fall kann die Diagnose der Lanze vollkommen automatisiert ablaufen. Bei einer Lanze mit Manipulator kann die Selbstdiagnose von einem übergeordneten Leitsystem (in einer genügend langen Prozesspause) gestartet werden. Der Manipulator steckt den Kurzschlussadapter auf die Lanze, woraufhin die Selbstdiagnose durchgeführt wird. Ist diese beendet, wird der Kurzschlussadapter automatisch wieder vom Manipulator abgenommen.

Da die Analyseeinheit genau weiß, welche Spannungen vom Digital/Analog-Umsetzer erzeugt werden, und in weiterer Folge am Analog/Digital-Wandler wieder gemessen werden sollen, kann eine Kalibrierung des Systems ebenfalls realisiert werden. In diesem Fall wird die gemessene Abweichung von Sollwertspannung zum Messspannungswert in der Analyseeinheit kompensiert. Diese Kalibrierung ist vorzugsweise anhand des Vergleichs des von der Analyseeinheit über dem Digital/Analog-Umsetzer in den zusätzlichen Schaltkreis eingespeisten Sollspannungswertes, und dem im ersten Schaltkreis gemessenen Messspannungswert vornehmbar. Bei der Kalibrierung wird eine Abweichung der Mess- und Probeentnahmelanze bestimmt, die in diesem Fall als "Normal" bezeichnet wird. Anschließend werden die ermittelten Abweichungen bei der darauffolgenden Benutzung der Mess- und Probeentnahmelanze zur Korrektur der Messspannungswerte herangezogen. Damit kann beispielsweise ein unnötiger Ausfall der Lanze durch Säuberung entfallen.

Die Mess- und Probeentnahmelanze kann mit einer Ablagevorrichtung zum Einlegen einer wie oben beschriebenen Mess- und Probeentnahmelanze zu einer Baueinheit weitergebildet werden, bei der die Elektronikeinheit zumindest einen Magnetschalter umfasst und die Ablagevorrichtung zumindest einen Magnet umfasst, durch welchen der Magnetschalter bei Einlegen der Mess- und Probeentnahmelanze (2,20) aktivierbar ist. Bevorzugt ist zudem ein Kurzschlussadapter vorgesehen, in welchen die Tauchsonde einsteckbar ist, d.h. die Lanze wird mit einem Magnetschalter im Elektronikgehäuse ausgerüstet. Das Gegenstück, der Magnet, befindet sich in der Ablagevorrichtung, wo auch der Kurzschlussadapter eingebaut ist. Wird die gesamte Lanze in diese Ablagevorrichtung eingelegt, wird dies über den Magnetschalter erkannt. Der Diagnosezyklus kann dadurch automatisiert gestartet werden.

Bevorzugt ist eine automatisierte Durchführung der Selbstdiagnose der Mess- und Probeentnahmelanze nach der Aktivierung des Magnetschalters bei Einlegen der Mess- und Probeentnahmelanze vorgesehen. D.h. die Analyseeinheit bringt den Schalter selbstständig in Y-Stellung, so dass die Selbstdiagnose starten kann.

Weitere Merkmale, Eigenschaften und Vorteile der vorliegenden Erfindung ergeben sich aus der nachfolgenden Beschreibung unter Bezugnahme auf die beiliegenden Figuren. Darin zeigen schematisch:
- FIG 1:: eine Mess- und Probeentnahmelanze zum Messen metallurgischer Kenngrößen und zum Ziehen einer Probe flüssiger Schmelze nach dem Stand der Technik,
- FIG 2:: ein erstes Beispiel einer erfindungsgemäßen Messund Probeentnahmelanze,
- FIG 3:: sehr schematisch das erfindungsgemäße Verfahren,
- FIG 4:: ein beispielhaftes Wegprofil aus Temperatur und Lanzenposition,
- FIG 5:: ein zweites Beispiel einer erfindungsgemäßen Messund Probeentnahmelanze mit zugeschaltetem Schaltkreis,
- FIG 6:: ein zweites Beispiel einer erfindungsgemäßen Messund Probeentnahmelanze mit weggeschaltetem Schaltkreis,
- FIG 7:: eine Ablagevorrichtung für eine Mess- und Probeentnahmelanze,
- FIG 8:: eine Selbstkalibrierung einer Mess- und Probeentnahmelanze.

Obwohl die Erfindung im Detail durch das bevorzugte Ausführungsbeispiel näher illustriert und beschrieben wurde, ist die Erfindung nicht durch die offenbarten Beispiele eingeschränkt. Variationen hiervon können vom Fachmann abgeleitet werden, ohne den Schutzumfang der Erfindung, wie er durch die nachfolgenden Patentansprüche definiert wird, zu verlassen.

Bei der Erzeugung und Verarbeitung von Flüssigmetallschmelzen, wie z.B. bei BOF, AOD, L TS, RH und CC müssen chemische Proben entnommen werden und physikalische Parameter wie z.B. die Temperatur bestimmt werden. Die gemessenen Werte sind für die weitere Bearbeitung immens wichtig, da die weiteren Bearbeitungsschritte davon abhängig sind als auch die Qualität des Stahls. Die Temperatur ist ein wesentlicher Eingabefaktor für die Prozessmodelle der Stranggussanlage.

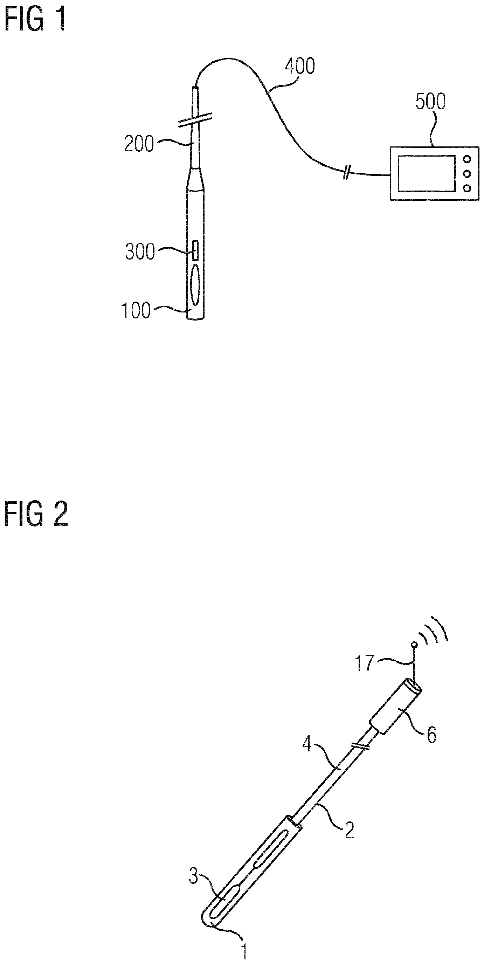

FIG 1 zeigt eine Mess- und Probeentnahmelanze 200 zum Messen metallurgischer Kenngrößen und zum Ziehen einer Probe flüssiger Schmelze nach dem Stand der Technik. Die Probenentnahme und Bestimmung der Temperatur wird üblicherweise mit einer Tauchsonde 100, welche auch als Einwegtauchsonde ausgeführt sein kann, durchgeführt. Die Tauchsonden 100 mit integrierten Sensorelementen werden auf die Lanze 200 aufgesteckt. Die Lanze 200 ist mit einem Kontaktstück 300 mit der Tauchsonde 100 verbunden. Ein Kabel 400 führt Signale aus der Tauchsonde 100 über das Kontaktstück 300 und der Lanze 200 zu einem Auswertegerät 500. Zwischen Lanze 200 und Auswertegerät 500 können auch Messverstärker und andere Bauteile verbaut werden.

Am Markt sind Lanzensysteme erhältlich, die eine drahtlose Übertragung der Messspannungswerte von der Lanze zum Auswertegerät erlauben. Diese sind so ausgeführt, dass die analogen Messspannungswerte kontinuierlich zum Auswertegerät übertragen werden. Die Verarbeitung der übertragenen Messspannungswerte findet dabei im Auswertegerät statt. Kommt es bei der kontinuierlichen Übertragung der Daten von der Lanze zum Auswertegerät zu Datenverlusten, ist eine Berechnung der Temperatur nicht möglich und das Messsystem verwirft den Vorgang. Es ist sodann eine neue Messsonde aufzustecken und eine neue Messung durchzuführen.

Das Problem der ungenauen Messungen durch den Bediener der Lanze wird momentan nicht durch technische Maßnahmen gelöst, sondern allenfalls durch Schulungen und allgemein qualitätssichernde Maßnahmen, wie z.B. das Vier-Augen-Prinzip.

Mit der Erfindung wird hier nun Abhilfe geschaffen. FIG 2 zeigt ein erstes Beispiel einer erfindungsgemäßen Mess- und Probeentnahmelanze 2. Auch hier sind Tauchsonden 1 mit integrierten Sensorelementen auf einer Lanze 2 aufgesteckt. Die Lanze 2 ist über ein Kontaktstück 3 mit der Tauchsonde 1 verbunden. In der Lanze 2 ist eine Elektronikeinheit 6 befestigt. In die Elektronikeinheit 6 wird das Kabel 4 vom Kontaktstück 3 geführt. Das Kabel 4 führt Signale aus der Tauchsonde 1 über das Kontaktstück 3 zur Elektronikeinheit 6. In der Elektronikeinheit 6 kann eine analoge Vorverarbeitung, wie beispielsweise Verstärkung, Filterung, durchgeführt werden.

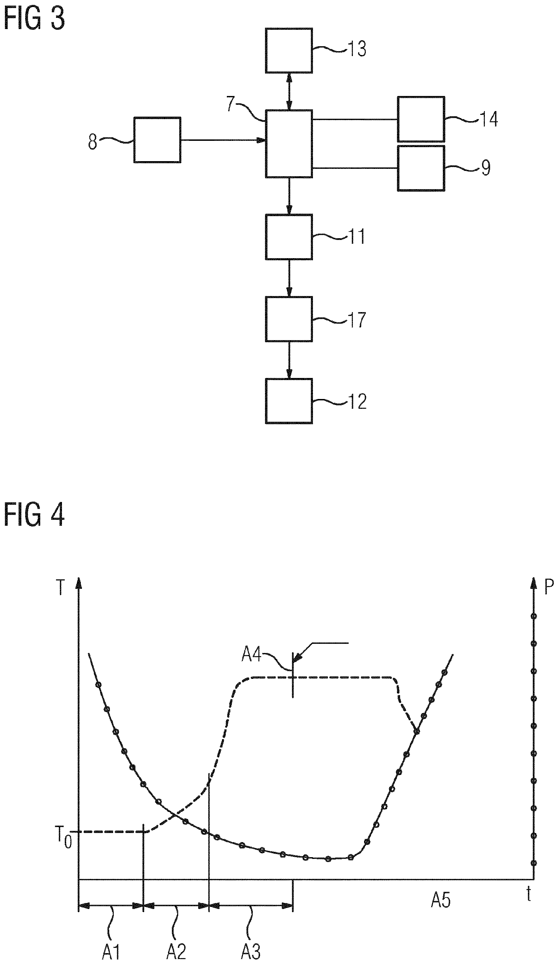

FIG 3 zeigt sehr schematisch das erfindungsgemäße Verfahren.

Das Signal wird in der Elektronikeinheit 6 (FIG 2) von einem A/D-Wandler 8 digitalisiert. Eine Analyseeinheit 7 verarbeitet das digitalisierte Signal und berechnet die physikalischen Parameter wie z.B. Temperatur, Sauerstoffgehalt und Kohlenstoffgehalt. Zusätzlich kann die Analyseeinheit 7 weitere Sensoren bei der Ermittlung der Parameter berücksichtigen. Jeder Messvorgang wird über eine Sendeeinheit 11 mit Antenne 17 an ein Automatisierungssystem 12 versendet. Die Übertragung der Daten kann z.B. über WirelessHART oder Industrial WLAN erfolgen. Eine Ausführung ist auch mit jeder anderen geeigneten Übertragungstechnik denkbar wie beispielsweise Bluetooth. Im Automatisierungssystem 12 werden die Daten weiter verarbeitet und z.B. anderen Automatisierungssystemen und/oder Prozessmodellen zugeführt und/oder einem Bediener angezeigt. In einer vorzugsweisen Ausführung der Erfindung werden die aufgezeichneten Daten der Sensoren der Tauchsonde und der Analyseeinheit in einem Speicher 13 gespeichert, der z.B. als Ringspeicher ausgeführt sein kann. Je nach Ausführung der Datenübertragung werden die aufgezeichneten Daten nach Abschluss der Übertragung des Messvorganges über dasselbe Medium versandt (z.B. WLAN). Wird der Messvorgang z.B. über WirelessHART versendet, können die Messdaten nachträglich über eine separate WLAN-Schnittstelle versendet werden. Genauso ist vorstellbar, dass die Messdaten nicht aktiv versandt werden, sondern passiv von einem IT-System aus dem Speicher 13 abgerufen werden. Beim passiven Abrufen fungiert die Lanze 2 (FIG 2) als Server und nicht als Client. Auf der Lanze 2 (FIG 2) und/oder der Elektronikeinheit 6 (FIG 2) können auch visuelle Zustandsindikatoren 14 angebracht werden. Die Zustandsindikatoren 14 können z.B. als Multi-Color LED ausgeführt werden. Je nach Status der Messung können die LED in verschiedenen Farben leuchten, wie nachfolgend beispielhaft anhand vom Zustand der Messung/Farbe aufgezeigt wird:
- Probe aufgesteckt und bereit zur Messung / Grün Dauerlicht,
- Messung läuft (Schlacke erkannt)/ Gelb blinkend,
- Messung läuft (Stahl erkannt) / Gelb Dauerlicht,
- Messung abgeschlossen und Lanze 2 (FIG 2) herausziehen / Rot Dauerlicht,
- Messung ungültig und Lanze 2 (FIG 2) herausziehen / Rot blinkend.

Um eine automatische Zuordnung der verschiedenen Lanzen 2 (FIG 2) zu einer Messposition zu ermöglichen, werden vorzugsweise entweder auf der Lanze 2 (FIG 2) eindeutige Identifizierungen angebracht (nicht gezeigt), wie beispielsweise Barcodes, QR-Codes, RFID-Tags, die drahtlos ausgelesen werden können oder die Lanze 2 (FIG 2) kann selbst eine Ortung vornehmen. So kann z.B. im Nahbereich des Konverters oder eines anderen Aggregats eine RFID-Antenne angebracht werden, die den auf der Lanze 2 (FIG 2) angebrachten RFID-Tag erkennt. Die Zuordnung von Lanze 2 (FIG 2) zu Aggregat erfolgt dann im Automatisierungssystem 12. Genauso ist vorstellbar, dass z.B. bei einem Stahlwerk mit drei Konvertern jeweils vor einem Konverter ein Industrial WLAN-Access Point angebracht wird.

Die Übertragungsreichweite reicht aus, damit an jeder Position vor den Konvertern ein Signal aller drei Access Points empfangbar ist. Über die Bestimmung der Empfangsstärke der jeweiligen Access Points an der Lanze 2 (FIG 2) kann die Elektronik ermitteln, welcher Access Point am nächsten ist und damit auch, vor welchem Konverter sie sich befindet. Bei der Verwendung von anderen Übertragungsmethoden, z.B. WirelessHART kann ebenfalls ermittelt werden, welchen Weg im (Mesh-)Netzwerk das Signal nimmt und dadurch eine grobe Position ermittelt werden.

Die Elektronikeinheit 6 (FIG 2) wird mit einem Beschleunigungssensor 9 ausgeführt. Über den Beschleunigungssensor 9 wird der Eintauchvorgang aufgezeichnet. Die Analyseeinheit 7 ermittelt aus dem aufgezeichneten Eintauchvorgang ein Wegprofil des Eintauchvorgangs der Tauchsonde 1 (FIG 2). FIG 4 zeigt ein beispielhaftes Wegprofil aus Temperatur T, wobei mit T₀ die Umgebungstemperatur gemeint ist, und Lanzenposition P, aufgetragen über die Zeit t. Die Zeit t ist dabei in 5 Abschnitte (Abschnitt A1-A5) unterteilt, welche den Prüfvorgang, ob eine Messung korrekt durchgeführt worden ist, darstellen:
Abschnitt A1: vor dem Eintauchen in die Schlacke ist eine gleichmäßige Abwärtsbewegung der Lanze 2 (FIG 2) messbar, während die Temperatur T der Umgebungstemperatur T₀ entspricht, also ungefähr gleich bleibt. Leichte Temperaturschwankungen können auch hier schon durch das Annähern der Lanze 2 (FIG 2) an die Oberfläche der Flüssigmetallschmelze auftreten.

Abschnitt A2: Die gemessene Temperatur der Tauchsonde steigt in der Schlacke nun erstmals signifikant an. Beim Auftreffen der Tauchsonde 1 (FIG 2) auf die Flüssigmetallschmelze ist eine Verzögerung der Geschwindigkeit der Lanze 2 (FIG 2) messbar, z.B. wie durch einen kurzen Ruck. Die Bewegung ist im Vergleich zu der außerhalb der Schlacke etwas langsamer.

Abschnitt A3: Beim Auftreffen der Tauchsonde 1 (FIG 2) auf die unter der Schlacke befindliche Metallschmelze (Stahl) ist ein erneuter kurzer Ruck, durch eine Veränderung der Dichte und somit des Auftriebs der auf die Lanze 2 (FIG 2) wirkt, festzustellen. Die Temperatur in der Metallschmelze ist wesentlich höher als die der Schlacke, aus diesem Grund steigt die Temperatur nun drastisch auf den der Flüssigmetallschmelze an.

Abschnitt A4: Stellt die Analyseeinheit 7 (FIG 2) fest, dass die Tauchsonde 1 (FIG 2) einen definierten Zeitraum, beispielsweise zwischen 3s und 10s, und/oder eine definierte Eintauchtiefe, beispielsweise 60 cm oder 1 m in die Metallschmelze aufweist, und/oder ein definierter Temperatursprung, beispielsweise 50°C im Vergleich zur Schlacke, erkennbar ist, wird der Messvorgang als gültig angesehen. Ansonsten wird der Messvorgang als ungültig gekennzeichnet. War der Messvorgang gültig, berechnet die Analyseeinheit 7 (FIG 3) einen Messerwert für die Temperatur und/oder den Sauerstoffgehalt und/oder den Kohlenstoffgehalt der Flüssigmetallschmelze. Sind die Messspannungswerte berechnet, werden diese gemeinsam über die Schnittstelle an das Automatisierungssystem 12 (FIG 3) gesendet.

Abschnitt A5: Ist der Messvorgang abgeschlossen (egal ob gültig oder nicht), wird dem Bedienpersonal akustisch, optisch oder über Vibration mitgeteilt, dass er die Lanze 2 (FIG 2) aus der Schmelze ziehen muss, um diese vor Beschädigungen zu bewahren.

Idealerweise fährt die Elektronik nach einem definierten Zeitraum, z.B. nach 1 min, nach dem Entfernen der Tauchsonde 1 (FIG 2) in einen Stromsparmodus bei dem Sonde 1, Sensoren (hier z.B. die Zustandsindikatoren 14) bzw. Antenne 17 (FIG 2) abgeschaltet werden oder in einen Low-Power-Modus versetzt werden. Der Stromsparmodus kann auch aktiviert werden, wenn über einen definierten Zeitraum kaum oder keine Bewegungen von einem Beschleunigungssensoren detektiert werden.

Die Elektronik prüft im Stromsparmodus von Zeit zu Zeit, ob eine Tauchsonde 1 (FIG 2) angesteckt wurde, beispielsweise durch eine Widerstandsmessung an dem Kontaktstück 3 (FIG 2) oder fragt die Beschleunigungsdaten ab. Wird festgestellt, dass die Lanze 2 (FIG 2) wieder benutzt wird, werden alle Bauteile aus dem Stromsparmodus geweckt und eine Netzwerkverbindung hergestellt.

Üblicherweise wird die Lanze 200 (FIG 1) des Stands der Technik von einem Bediener oder auch Manipulator in die Flüssigmetallschmelze getaucht. Bei diesem Eintauchvorgang wird die Tauchsonde 100 (FIG 1) des Stands der Technik zerstört (verbrannt). Wird die Lanze 200 (FIG 1) des Stands der Technik nach Beendigung des Messvorganges nicht schnell genug aus der Flüssigmetallschmelze gezogen, können die Kontakte des Kontaktstücks 300 stark verschmutzt oder sogar zerstört werden. Wird das Equipment nicht in regelmäßigen Zeitintervallen auf korrekte Funktion hin überprüft, liefert die Lanze falsche Messergebnisse. Die momentan im Einsatz befindlichen Messlanzen 200 des Stands der Technik werden durch Aufstecken eines eigens dafür vorgesehenen Messadapters auf ihre korrekte Funktion überprüft. Hierfür wird der Messadapter von Hand also von einer Person manuell anstelle einer Tauchsonde 100 (FIG 1) auf das Kontaktstück 300 (FIG 1) aufgesteckt. Anschließend werden von diesem Messadapter bestimmte vorgebare Temperaturen simuliert und über das Kontaktstück 300 (FIG 1), und Kabel 400 (FIG 1) an das Auswertegerät 500 (FIG 1) weitergegeben. Werden diese Temperaturen am Auswertegerät 500 (FIG 1) korrekt wiedergegeben, so liegen im Messkreis keine Fehler vor. Liegt eine Abweichung vor, muss das System gereinigt, oder das Kontaktstück 300 (FIG 1) ausgetauscht werden. Eine Neu-Kalibrierung der Elektronik ist nicht möglich. Diese unbedingt notwendigen Funktionsüberprüfungen werden nicht oder nur unzuverlässig oft durchgeführt. Daraus resultieren unweigerlich unerkannte Falschmessungen.

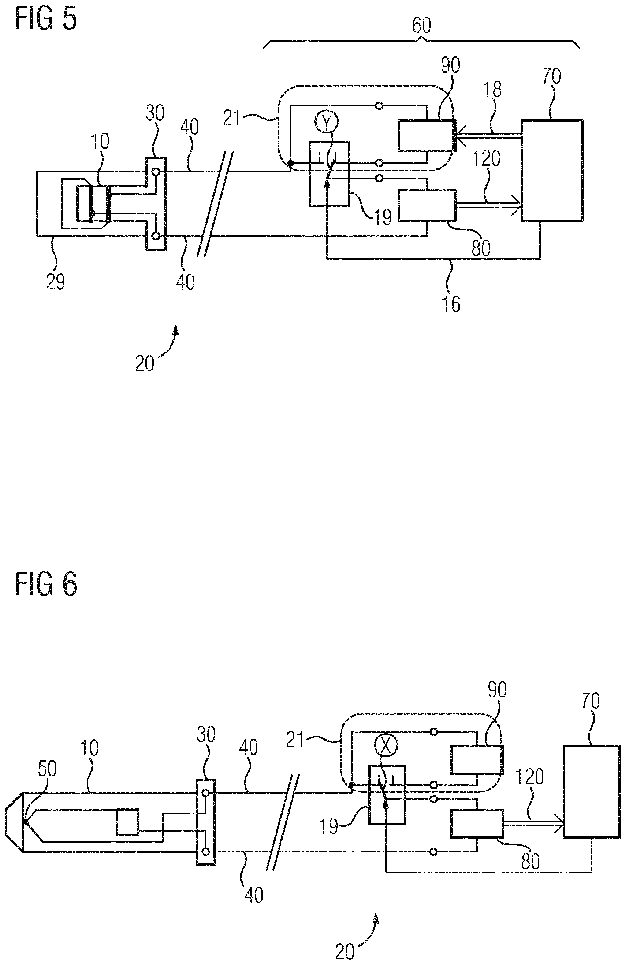

FIG 5 zeigt nun ein zweites Beispiel einer erfindungsgemäßen Mess- und Probeentnahmelanze 20 mit zugeschaltetem Schaltkreis 21, durch welchen nun eine Analyse-Elektronik vollständig in die Messlanze 20 integriert ist. Mithilfe dieser Analyse-Elektronik kann sich die Messlanze 20 selbst diagnostizieren und auch kalibrieren.

Der Elektronikeinheit 60 wird durch den zugeschalteten Schaltkreis 21 ein Digital/Analog-Umsetzer 90 hinzugefügt. Dieser Digital/Analog-Umsetzer 90 ist ein hochgenauer Spannungsgenerator, mit welchem die in der Tauchsonde 10 enthaltenen Sensoren, welche Spannungen liefern, simuliert werden können. Die Mess- und Probeentnahmelanze 20 weist nun zwei Betriebsmodi auf, den Selbstdiagnosemodus und den Messmodus: In dem Selbstdiagnosemodus wird in den zusätzlichen Schaltkreis 21 ein Spannungsgenerator eingekoppelt. Mit diesem werden die Sensorspannungen einer Tauchsonde 10 simuliert. In dieser Betriebsart befindet sich der Schalter 19 in Y-Stellung. Die Umschaltung 16 kann automatisch durch die Analyseeinheit 70 erfolgen.
Die Spannungsquelle wird hier zwar als Digital/Analog-Übersetzter 90 ausgeführt, es kann aber auch jede andere geeignete Spannungsquelle herangezogen werden. Die Analyseeinheit 70 gibt dem Digital/Analog-Übersetzer 90 zu erzeugende Sollspannungswerte 18 für die Testspannung vor. Diese werden in weiterer Folge durch die Y-Stellung des Schalters 19 in Serie als Spannungen in den Messkreis eingekoppelt. Über die Kabel 40 werden die Sollspannungswerte 18 an das Kontaktstück 30 weitergegeben, wo sie über den Kurzschlussadapter 29 direkt wieder zurück an den Analog/Digital-Wandler 80 der Lanze 20 zurückgeführt und somit gemessen werden können. Fehler im Messkreis können von der Analyseeinheit 70 somit erkannt und darauf reagiert werden. So werden die Messspannungswerte in den Analog/Digital-Wandler 80 digitalisiert und die digitalisierten Messspannungswerte 120 an die Analyseeinheit 70 gesendet.

Der Analog/Digital-Wandler 80 misst daher die Spannung, welche vom Digital/Analog-Umsetzer 90 erzeugt wurden. Treten irgendwo im Messkreis Spannungsverluste in Form von erhöhten Übergangswiderständen oder durch Leitungsbruch auf, kann dies durch den Analog/Digital-Wandler 80 erkannt werden. Dabei sind beispielsweise erhöhte Übergangswiderstände durch ein verschmutztes Kontaktstück zu erwarten. Auch können Leitungs- oder Kabelbrüche auftreten, wenn die Tauchsonde 10 zu lange in der Flüssigmetallschmelze eingetaucht und somit komplett verbrannt wurde. In diesem Fall ist von der vollständigen Zerstörung von Kontaktstück 30, aber auch des Kabels 40 auszugehen. Auch kann Kabelbruch auftreten, wenn das Kabel 40 durch andauernde Beanspruchung reißt.

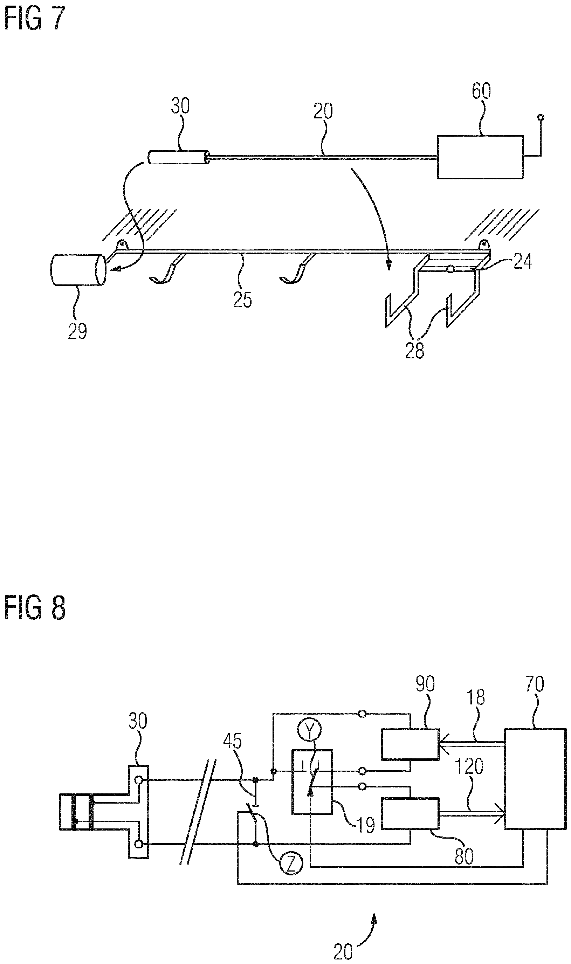

Es ist vorteilhafterweise keine Falschmessungen durch ein verschmutztes oder schlecht kalibriertes Messsystem mehr vorhanden, da die Lanze 20 (FIG 5,6) nach jedem Einsatz automatisch eine Selbstdiagnose durchführt und die Messlanze 20 (FIG 5,6) sich bei geringem Abweichungen selbst kalibriert. Ist eine Selbstkalibrierung nicht mehr möglich, wird das Bedienpersonal automatisch informiert, dass das Kontaktstück 30 gewechselt oder gereinigt werden muss. Das Bedienpersonal wird entlastet, da die Diagnose des Messkreises von der Lanze 20 selbst durchgeführt wird. Es sind zudem keine unerwarteten Ausfälle durch beschädigte Kontaktstücke 30 da, welche erst zu spät erkannt werden. Die Diagnose kann in jeder Prozesspause automatisch durchgeführt werden. Die zur Kalibrierung der Lanze 20 (FIG 5,6) notwendigen elektronischen Komponenten sind zudem bereits in der Messlanze 20 selbst integriert; dadurch entfällt der aufwändige separate Messadapter, mit welchem der Bediener die Lanze 20 (FIG 5,6) kalibrieren muss. Da die Lanze 20 (FIG 5,6) nach jedem Messvorgang in einem speziell vorgesehenen Platz abgelegt werden muss, in welchem z.B. der Kurzschlussadapter 30 integriert ist (FIG 7), sind die vor Verschmutzung anfälligen Kontakte der Lanze 20 (FIG 5,6) immer geschützt.

Es kann ein sequentieller Test von mehreren verschiedenen Spannungen für jeden einzelnen in der Tauchsonde 10 eingebauten Sensor, mit welchen hintereinander der Messkreis überprüft wird, stattfinden.

Auch kann die Analyseeinheit 70 automatisch den Schalter 19 in die notwendige Y-Stellung bringen.

FIG 6 zeigt das zweite Beispiel der erfindungsgemäßen Mess- und Probeentnahmelanze 20 mit weggeschalteten Schaltkreis. Die Mess- und Probeentnahmelanze 20 weist nun den Messmodus auf: in diesem Modus werden wie gewohnt Tauchsonden 10 auf die Lanze 20 aufgesteckt. Die Elektronik erfasst die Sensorwerte, gibt sie an die Analyseeinheit 70 weiter, welche sie aufbereitet und einem Leitsystem zur Verfügung stellt. Der Schalter 19 ist in X-Stellung, wodurch die von der Tauchsonde 10 generierten Sensor-Spannungssignale über das Kontaktstück 30 und die Signalkabel 40 direkt an den Analog/DigitalWandler 80 der Messelektronik geleitet werden. Der Analog/Digital-Wandler 80 wandelt die Spannungssignale, beispielsweise von einem in der Tauchsonde 10 befindlichen Thermoelement 50, in einen digitalen Messwert 120 und gibt diesen an die Analyseeinheit 70 weiter.

Bevorzugt bringt die Analyseeinheit 70 automatisch den Schalter 19 in die notwendige X-Stellung oder Y-Stellung (FIG 5). In diesem Fall kann die Diagnose der Lanze 20 vollkommen automatisiert ablaufen.

FIG 7 zeigt eine Ablagevorrichtung 25 für eine Mess- und Probeentnahmelanze 20. Die Lanze 20 wird mit einem Magnetschalter (nicht gezeigt) im Elektronikgehäuse 60 ausgerüstet. Das Gegenstück, der Magnet 24, befindet sich in der Ablagevorrichtung 25, wo auch der Kurzschlussadapter 29 eingebaut ist. Dabei kann der Magnet 24 zwischen zwei Haken 28 angebracht werden, auf die die Elektronikeinheit 60 aufgelegt wird. Das Kontaktstück 30 wird dabei bei Einlegen der Lanze 20 in den Kurzschlussadapter 29 eingesteckt. Auch können noch weitere Haken und eine Wandhalterung zum Befestigen der Ablagevorrichtung 25 an der Wand vorhanden sein. Wird die gesamte Lanze 20 in diese Ablagevorrichtung 25 eingelegt, wird dies über den Magnetschalter erkannt. Der Diagnosezyklus kann dadurch automatisiert gestartet werden. Ein manueller Start der Selbstdiagnose durch einen Knopf an der Lanze, welchen der Bediener der Lanze 20 drücken kann, ist ebenfalls möglich.

FIG 8 zeigt eine Selbstkalibrierung einer erfindungsgemäßen Mess- und Probeentnahmelanze 20.

Da die Analyseeinheit 70 genau weiß, welche Spannungen vom Digital/Analog-Umsetzer 90 erzeugt werden, und in weiterer Folge am Analog/Digital-Wandler 80 wieder gemessen werden sollen, kann eine Kalibrierung des Systems ebenfalls realisiert werden. In diesem Fall wird die gemessene Abweichung von Sollspannungswert 18 zum Messspannungswert 120 in der Analyseeinheit 70 kompensiert. Bei einer Selbstkalibrierung des Systems wird weder eine Sonde, noch der Kurzschlussadapter 29 (FIG 6) an die Lanze aufgesteckt. Das Kontaktstück 30 bleibt offen (siehe obige Abbildung). Es wird ein Schalter 45 geschlossen (Z-Stellung), dadurch wird intern der Schaltkreis geschlossen. Da in diesem Kreis keine Verschmutzungen auftreten können, muss die gemessene Spannung am A/D-Wandler 80 gleich der am Ausgang vom D/A-Umsetzer 90 sein. Ist dies nicht der Fall, kann der Spannungsausgang des D/A-Umsetzers 90 so angepasst werden, dass wieder die richtige Spannung am A/D-Wandler 80 gemessen wird. Idealerweise wird die Selbstkalibrierung vom System selbst gestartet.

## Patentansprüche

1. Mess- und Probeentnahmelanze (2,20) zum Messen metallurgischer Kenngrößen und zum Ziehen einer Probe flüssiger Schmelze,
wobei die Mess- und Probeentnahmelanze (2,20) ein Kontaktstück (3,30) und eine lösbare am Kontaktstück (3,30) befestigbare Tauchsonde (1,10) umfasst,
wobei die Tauchsonde (1,10) eintauchbar ist und zumindest einen Sensor zur Ausgabe eines für die Schmelze charakteristischen Sensorsignals aufweist,
**dadurch gekennzeichnet, dass** die Mess- und Probenentnahmelanze (2,20) ein Gehäuse mit einer Elektronikeinheit (6, 60) aufweist, insbesondere an der sensorabgewandten Seite der Mess- und Probeentnahmelanze (2,20),
wobei der Sensor über das Kontaktstück (3,30) mit dem Gehäuse durch eine elektrische Verbindung verbunden ist, sodass das Sensorsignal über die elektrische Verbindung zu der Elektronikeinheit (6,60) führbar ist, und
wobei die Elektronikeinheit (6,60) zumindest eine analoge Verarbeitungseinheit zum anlogen Vorverarbeiten des Sensorsignals umfasst,
wobei die Elektronikeinheit (6,60) einen Beschleunigungssensor (9) zum Aufzeichnen eines Eintauchvorgangs umfasst und die Elektronikeinheit (6,60) dazu eingerichtet ist, über den Beschleunigungssensor (9) den Eintauchvorgang aufzuzeichnen.

2. Mess- und Probeentnahmelanze (2,20) nach Anspruch 1,
**dadurch gekennzeichnet, dass** die Elektronikeinheit (6,60) dazu eingerichtet ist, aus dem aufgezeichneten Eintauchvorgang ein Wegprofil des Eintauchvorgangs zu erstellen.

3. Mess- und Probeentnahmelanze (2,20) nach einem der vorhergehenden Ansprüche,
**dadurch gekennzeichnet, dass** die Elektronikeinheit (6,60) einen Speicher (13) umfasst, insbesondere einen Ringspeicher.

4. Mess- und Probeentnahmelanze (2,20) nach einem der vorhergehenden Ansprüche,
**dadurch gekennzeichnet, dass** auf der Mess- und Probeentnahmelanze (2,20) und/oder an dem Gehäuse Zustandsindikatoren (14) angeordnet sind.

5. Mess- und Probeentnahmelanze (2,20) nach Anspruch 4,
**dadurch gekennzeichnet, dass** die Zustandsindikatoren (14) visuell sind, und der Zustand, in welchem sich die Mess- und Probeentnahmelanze (2,20) befindet, durch einen Farbcode anzeigbar ist.

6. Mess- und Probeentnahmelanze (2,20) nach einem der vorhergehenden Ansprüche,
**dadurch gekennzeichnet, dass** nach einem vorab festgelegten Zeitraum nach Entfernen der Tauchsonde (1,10) die Elektronikeinheit (6,60) in einen Stromsparmodus versetzbar ist und bei Feststellen der Benutzung der Mess- und Probeentnahmelanze (2,20) durch die Elektronikeinheit (6,60) der Stromsparmodus aufhebbar ist.

7. Mess- und Probeentnahmelanze (2,20) nach einem der vorhergehenden Ansprüche,
**dadurch gekennzeichnet, dass** entweder Hilfsmittel zur eindeutigen drahtlosen Identifizierung der Mess- und Probeentnahmelanze (2,20) auf die Mess- und Probeentnahmelanze (2,20) aufgebracht sind und/oder dass durch die Mess- und Probeentnahmelanze (2,20) selbst eine Ortung vornehmbar ist.

8. Mess- und Probeentnahmelanze (20) nach einem der vorhergehenden Ansprüche,
**dadurch gekennzeichnet, dass** die Elektronikeinheit (60) als einen ersten Schaltkreis einen Wandler zum Digitalisieren des Sensorsignals und eine Analyseeinheit (70) umfasst und einen zusätzlichen Schaltkreis (21) zur Durchführung einer Selbstdiagnose der Mess- und Probeentnahmelanze (20) umfasst, wodurch der zusätzliche Schaltkreis (21) durch einen Schalter (19) in den ersten Schaltkreis zu- und wegschaltbar ist, und der zusätzliche Schaltkreis (21) einen Spannungsgenerator, insbesondere einen Digital-Analog-Umsetzer (90), umfasst.

9. Mess- und Probeentnahmelanze (20) nach Anspruch 8,
**dadurch gekennzeichnet, dass** die Mess- und Probenentnahmelanze (2, 20) einen Kurzschlussadapter (29) aufweist, in welchen die Tauchsonde (10) einsteckbar ist, und die Analyseeinheit (70) dazu eingerichtet ist, bei Zuschalten des zusätzlichen Schaltkreises (21) dem Spannungsgenerator zumindest einen vorgegebenen Spannungssollwert zuzuführen, welchen der Spannungsgenerator in den zusätzlichen Schaltkreis einbringt und wobei der Spannungssollwert durch eine elektrische Verbindung über das Kontaktstück (3,30) an den Kurzschlussadapter (29) führbar ist und anschließend über eine elektrische Verbindung an den Wandler zum Digitalisieren als Messspannungswert zurückführbar und dort digitalisierbar ist, wobei der digitalisierte Messspannungswert der Analyseeinheit (70) zuführbar ist.

10. Mess- und Probeentnahmelanze (20) nach einem der vorhergehenden Ansprüche 8 oder 9,
**dadurch gekennzeichnet, dass** bei Zuschalten des zusätzlichen Schaltkreises (21) durch die Analyseeinheit (70) dem Spannungsgenerator mehrere verschiedene Spannungssollwerte (18) zuführbar sind.

11. Mess- und Probeentnahmelanze (20) nach Anspruch 10,
**dadurch gekennzeichnet, dass** die Tauchsonde (10) mehrere Tauchsondensensoren aufweist, welche durch die mehreren verschiedenen Spannungssollwerte (18) diagnostizierbar sind.

12. Mess- und Probeentnahmelanze (20) nach einem der vorhergehenden Ansprüche 8-11,
**dadurch gekennzeichnet, dass** durch die Analyseeinheit (70) der zusätzliche Schaltkreis (21) durch den Schalter (19) automatisiert in den ersten Schaltkreis zu- und wegschaltbar ist.

13. Mess- und Probeentnahmelanze (20) nach einem der vorhergehenden Ansprüche 8-12,
**dadurch gekennzeichnet, dass** das anhand des Vergleichs des von der Analyseeinheit (70) über dem Digital/Analog-Umsetzer (90) in den zusätzlichen Schaltkreis (21) vorgegebenen Spannungssollwertes (18) und einem im ersten Schaltkreis gemessenen Messspannungswert (120) eine Kalibrierung vornehmbar ist.

14. Baueinheit aus einer Mess- und Probeentnahmelanze (2,20) nach einem der vorhergehenden Ansprüche und einer Ablagevorrichtung (25) zum Einlegen einer solchen Mess- und Probeentnahmelanze (2,20), wobei das Elektronikgehäuse (6,60) zumindest einen Magnetschalter umfasst und die Ablagevorrichtung zumindest einen Magnet umfasst, durch welchen der Magnetschalter bei Einlegen der Mess- und Probeentnahmelanze (2,20) aktivierbar ist.

15. Baueinheit nach Anspruch 14,
**dadurch gekennzeichnet, dass** die Baueinheit einen Kurzschlussadapter (29) aufweist, in welchen die Tauchsonde (1,10) einsteckbar ist.

16. Baueinheit nach Anspruch 14,
**dadurch gekennzeichnet, dass** die Baueinheit einen Ladeadapter aufweist, mit welchem die Elektronikeinheit (6, 60) der Mess- und Probeentnahmelanze (2,20) aufladbar ist.

17. Baueinheit nach einem der vorhergehenden Ansprüche 14-16,
**dadurch gekennzeichnet, dass** eine automatisierte Durchführung einer Selbstdiagnose der Mess- und Probeentnahmelanze (20) nach der Aktivierung des Magnetschalters bei Einlegen der Mess- und Probeentnahmelanze (20) vornehmbar ist.

18. Verfahren zum Messen metallurgischer Kenngrößen und zum Ziehen einer Probe flüssiger Schmelze mit einer Mess- und Probeentnahmelanze (2,20) mit einem Kontaktstück (3,30) und einer lösbaren am Kontaktstück (3,30) befestigbaren Tauchsonde (1,10), welche zumindest einen Sensor zur Ausgabe eines für die Schmelze charakteristischen Sensorsignals aufweist,
wobei die Tauchsonde (1,10) in die flüssige Schmelze zumindest teilweise eingetaucht wird, wobei der Sensor das für die Schmelze charakteristische Signal ausgibt,
**dadurch gekennzeichnet, dass** die Mess- und Probeentnahmelanze (2, 20) ein Gehäuse mit einer Elektronikeinheit (6, 60) aufweist, in welcher eine analoge Verarbeitungseinheit angeordnet ist, durch die das Sensorsignal zumindest analog vorverarbeitet wird,
wobei der Sensor über das Kontaktstück (3, 30) mit dem Gehäuse durch eine elektrische Verbindung verbunden ist, sodass das Sensorsignal über die elektrische Verbindung zu der Elektronikeinheit (6, 60) geführt wird, und der Eintauchvorgang durch einen Beschleunigungssensor (9) in der Elektronikeinheit (6,60) aufgezeichnet wird.

19. Verfahren zum Messen nach Anspruch 18,
**dadurch gekennzeichnet, dass**
durch den durch die Elektronikeinheit (6,60) aufgezeichneten Eintauchvorgang ein Wegprofil des Eintauchvorgangs erstellt wird und anhand dieses Wegprofils der Eintauchvorgang als gültig oder ungültig eingestuft wird.

20. Verfahren zum Messen nach einem der vorhergehenden Ansprüche 18-19,
**dadurch gekennzeichnet, dass**
durch auf der Mess- und Probeentnahmelanze (2,20) und/oder an dem Gehäuse angeordnete Zustandsindikatoren (14) der Zustand, in welchem sich die Mess- und Probeentnahmelanze (2,20) befindet, durch einen Farbcode angezeigt wird.

21. Verfahren zum Messen nach einem der vorhergehenden Ansprüche 18-20,
**dadurch gekennzeichnet, dass**
die Elektronikeinheit (60) in einem ersten Schaltkreis einen Wandler zum Digitalisieren des Sensorsignals und eine Analyseeinheit (70) umfasst und mit einem zusätzlichen Schaltkreis (21) umfassend einen Spannungsgenerator, insbesondere ein Digital-Analog-Umsetzer (90), eine Selbstdiagnose der Mess- und Probeentnahmelanze (20) durchgeführt wird, indem der zusätzliche Schaltkreis (21) durch den Schalter (19) in den ersten Schaltkreis zu- und weggeschaltet wird.

22. Verfahren zum Messen nach Anspruch 21,
**dadurch gekennzeichnet, dass**
die Tauchsonde (10) in einen Kurzschlussadapter (29) einsteckbar ist und bei Zuschalten des zusätzlichen Schaltkreises (21) die Analyseeinheit (70) dem Spannungsgenerator zumindest einen vorgegebenen Spannungssollwert (18) zuführt, welchen der Spannungsgenerator in den zusätzlichen Schaltkreis (21) einbringt und wobei der Spannungssollwert (18) durch eine elektrische Verbindung über das Kontaktstück (30) an den Kurzschlussadapter (29) zugeführt wird und anschließend über eine elektrische Verbindung an den Wandler als Messspannungswert zurückgeführt und dort digitalisiert wird, wobei der digitalisierte Messspannungswert (120) der Analyseeinheit (70) zugeführt wird.

23. Verfahren zum Messen nach Anspruch 21 oder 22,
**dadurch gekennzeichnet, dass**
beim Zuschalten des zusätzlichen Schaltkreises (21) durch die Analyseeinheit (70) dem Spannungsgenerator mehrere verschiedene Spannungssollwerte (18) zugeführt werden.

24. Verfahren zum Messen nach einem der Ansprüche 21-23,
**dadurch gekennzeichnet, dass**
durch die Analyseeinheit (70) der zusätzliche Schaltkreis (21) durch den Schalter (19) automatisiert in den ersten Schaltkreis zu- und weggeschaltet wird.

25. Verfahren zum Messen nach einem der vorhergehenden Ansprüche 21-24,
**dadurch gekennzeichnet, dass**
das anhand des Vergleichs des von der Analyseeinheit (70) über dem Spannungsgenerator in den zusätzliche Schaltkreis vorgegebenen Spannungssollwertes (18), und einem im ersten Schaltkreis gemessenen und digitalisierten Messspannungswert (120) eine Kalibrierung vorgenommen wird.

## Claims

1. Measuring and sampling lance (2, 20) for measuring metallurgical characteristic values and for taking a sample of liquid melt,
wherein the measuring and sampling lance (2, 20) incorporates a contact piece (3, 30) and an immersion probe (1, 10) which can be removably fastened to the contact piece (3, 30),
wherein the immersion probe (1, 10) can be immersed and has at least one sensor for the output of a sensor signal which is characteristic of the melt,
**characterised in that** the measuring and sampling lance (2, 20) has a housing with an electronic unit (6, 60), in particular onto the side of the measuring and sampling lance (2, 20) opposite to the sensor,
wherein the sensor is connected to the housing via the contact piece (3, 30) through an electrical connection, so that the sensor signal can be fed through the electrical connection to the electronic unit (6, 60), and
wherein the electronic unit (6, 60) incorporates at least one analogue processing unit for the analogue pre-processing of the sensor signal,
wherein the electronic unit (6, 60) incorporates an acceleration sensor (9) for recording an immersion operation, and the electronic unit (6, 60) is equipped for recording the immersion operation via the acceleration sensor (9).

2. Measuring and sampling lance (2, 20) according to claim 1, **characterised in that**
the electronic unit (6, 60) is equipped for producing a path profile for the immersion operation from the recorded immersion operation.

3. Measuring and sampling lance (2, 20) according to one of the preceding claims,
**characterised in that**
the electronic unit (6, 60) incorporates a memory (13), in particular a ring memory.

4. Measuring and sampling lance (2, 20) according to one of the preceding claims,
**characterised in that**
arranged on the measuring and sampling lance (2, 20) and/or on the housing are status indicators (14).

5. Measuring and sampling lance (2, 20) according to claim 4, **characterised in that**
the status indicators (14) are visual and the status, in which the measuring and sampling lance (2, 20) currently finds itself, can be indicated by colour code.

6. Measuring and sampling lance (2, 20) according to one of the preceding claims,
**characterised in that**
after a previously defined period of time after the withdrawal of the immersion probe (1, 10), the electronic unit (6, 60) can be switched into a current saving mode, and this current saving mode can be lifted when it is determined by the electronic unit (6, 60) that the measuring and sampling lance (2, 20) is being used.

7. Measuring and sampling lance (2, 20) according to one of the preceding claims,
**characterised in that**
either there are affixed onto the measuring and sampling lance (2, 20) facilities for the unambiguous wireless identification of the measuring and sampling lance (2, 20) and/or the measuring and sampling lance (2, 20) is itself able to establish its location.

8. Measuring and sampling lance (20) according to one of the preceding claims
**characterised in that**
the electronic unit (60) incorporates as a first electrical circuit a converter for digitising the sensor signal and an analysis unit (70), and an additional electrical circuit (21) for carrying out a self-diagnosis of the measuring and sampling lance (20), whereby the additional electrical circuit (21) can be switched into and out of the first electrical circuit by means of a switch (19), and the additional electrical circuit (21) incorporates a voltage generator, in particular a digital-to-analogue converter (90).

9. Measuring and sampling lance (20) according to claim 8,
**characterised in that**
the measuring and sampling lance (2, 20) has a short-circuit adapter (29), into which the immersion probe (10) can be plugged, and the analysis unit (70) is equipped for the purpose of feeding to the voltage generator, when the additional electric circuit (21) is switched in, at least one predefined voltage setpoint value, which the voltage generator introduces into the additional electrical circuit and wherein the voltage setpoint value can be fed through an electrical connection via the contact piece (3, 30) to the short-circuit adapter (29) and can then be fed back via an electrical connection to the converter for digitisation as a measurement voltage value where it can be digitised, wherein the digitised measurement voltage value can be fed to the analysis unit (70).

10. Measuring and sampling lance (20) according to one of the preceding claims 8 or 9,
**characterised in that**
when the additional electrical circuit (21) is switched in, several different voltage setpoint values (18) can be fed to the voltage generator by the analysis unit (70).

11. Measuring and sampling lance (20) according to claim 10,
**characterised in that**
the immersion probe (10) has several immersion probe sensors which can be diagnosed by the several different voltage setpoint values (18).

12. Measuring and sampling lance (20) according to one of the preceding claims 8-11,
**characterised in that**
the analysis unit (70) can, using the switch (19), automatically switch the additional electrical circuit (21) into and out of the first electrical circuit.

13. Measuring and sampling lance (20) according to one of the preceding claims 8-12,
**characterised in that**
by reference to a comparison of the prescribed voltage setpoint value (18), issued by the analysis unit (70) into the additional electrical circuit (21) via the digital-to-analogue converter (90), and a measurement voltage value (120) measured in the first electrical circuit, it is possible to carry out a calibration.

14. Assembly unit comprising a measuring and sampling lance (2, 20) according to one of the preceding claims and a deposit facility (25) for laying in such a measuring and sampling lance (2, 20), wherein the electronic housing (6, 60) incorporates at least one magnetic switch and the deposit facility (25) incorporates at least one magnet (24) by which the magnetic switch can be activated when the measuring and sampling lance (2, 20) is laid in.

15. Assembly unit according to claim 14,
**characterised in that**
the assembly unit has a short-circuit adapter (29), into which the immersion probe (1, 10) can be plugged.

16. Assembly unit according to claim 14,
**characterised in that**
the assembly unit has a charging adapter, using which the electronic unit (6, 60) of the measuring and sampling lance (20) can be charged up.

17. Assembly unit according to one of the preceding claims 14-16,
**characterised in that**
an automated performance of a self-diagnosis of the measuring and sampling lance (20) can be performed after the magnetic switch is activated when the measuring and sampling lance (20) is laid in.

18. Method for measuring metallurgical characteristic variables and for extracting a sample of liquid melt using a measuring and sampling lance (2, 20) with a contact piece (3, 30) and a releasable immersion probe (1, 10) which can be attached to the contact piece (3, 30), which has at least one sensor, for the output of a sensor signal characteristic of the melt,
wherein the immersion probe (1, 10) is immersed at least partially into the liquid melt, wherein the sensor outputs the signal characteristic of the melt,
**characterised in that**
the measuring and sampling lance (2, 20) has a housing with an electronic unit (6, 60), in which an analogue processing unit is arranged, by means of which the sensor signal is preprocessed, at least in analogue form,
wherein the sensor is connected to the housing by an electrical connection via the contact piece (3, 30), so that the sensor signal is fed via the electrical connection to the electronic unit (6, 60), and
the immersion operation is recorded by an acceleration sensor (9) in the electronic unit (6, 60).

19. Method for measuring according to claim 18,
**characterised in that**
using the immersion operation recorded by the electronic unit (6, 60) a path profile is generated for the immersion operation and, by reference to this path profile, the immersion operation is categorised as valid or invalid.

20. Method for measuring according to one of the preceding claims 18-19,
**characterised in that**
the status in which the measuring and sampling lance (2, 20) currently finds itself, is indicated by status indicators (14), arranged on the measuring and sampling lance (2, 20) and/or on the housing, by a colour code.

21. Method for measuring according to one of the preceding claims 18-20,
**characterised in that**
the electronic unit (60) incorporates, in a first electrical circuit, a converter for digitising the sensor signal and an analysis unit (70), and using an additional electrical circuit (21) incorporating a voltage generator, in particular a digital-to-analogue converter (90), a self-diagnosis of the measuring and sampling lance (20) is carried out, **in that** the additional electrical circuit (21) is switched into and out of the first electrical circuit by the switch (19).

22. Method for measuring according to claim 21,
**characterised in that**
the immersion probe (10) can be plugged into a short-circuit adapter (29), and when the additional electrical circuit (21) is switched in the analysis unit (70) feeds to the voltage generator at least one predefined voltage setpoint value (18) which the voltage generator introduces into the additional electrical circuit (21) and wherein the voltage setpoint value (18) is fed to the short-circuit adapter (29) via an electrical connection through the contact piece (30) and thereafter is fed back through an electrical connection as a measurement voltage value to the converter, and there it is digitised, wherein the digitised measurement voltage value (120) is fed to the analysis unit (70).

23. Method for measuring according to claim 21 or 22,
**characterised in that**
when the additional electrical circuit (21) is switched in, several different voltage setpoint values (18) are fed to the voltage generator by the analysis unit (70).

24. Method for measuring according to one of the claims 21-23, **characterised in that**
using the switch (19), the analysis unit (70) automatically switches the additional electrical circuit (21) into and out of the first electrical circuit.

25. Method for measuring according to one of the claims 21-24, **characterised in that**
by reference to the comparison of the prescribed voltage setpoint value (18), issued by the analysis unit (70) into the additional electrical circuit via the voltage generator, and a measurement voltage value (120) measured and digitised in the first electrical circuit, a calibration is carried out.

## Revendications

1. Lance de mesure et de prélèvement d'échantillons (2, 20) pour la mesure de grandeurs caractéristiques métallurgiques et pour la prise d'un échantillon de matière fondue liquide,
la lance de mesure et de prélèvement d'échantillons (2, 20) comportant une pièce de contact (3, 30) et une sonde à immersion (1, 10) pouvant être fixée de manière amovible sur la pièce de contact (3, 30),
dans laquelle la sonde à immersion (1, 10) peut être immergée et comprend au moins un capteur pour l'émission d'un signal de capteur caractéristique de la matière fondue,
**caractérisée en ce que** la lance de mesure et de prélèvement d'échantillons (2, 20) comprend un boîtier muni d'une unité électronique (6, 60), en particulier sur le côté opposé au capteur de la lance de mesure et de prélèvement d'échantillons (2, 20),
dans laquelle le capteur est relié par l'intermédiaire de la pièce de contact (3, 30) au boîtier par l'intermédiaire d'une liaison électrique de telle sorte que le signal de capteur puisse être guidé par l'intermédiaire de la liaison électrique jusqu'à l'unité électronique (6, 60), et
dans laquelle l'unité électronique (6, 60) comporte au moins une unité de traitement analogique pour le prétraitement analogique du signal de capteur,
dans laquelle l'unité électronique (6, 60) comporte un capteur d'accélération (9) pour l'enregistrement d'un processus d'immersion et l'unité électronique (6, 60) est conçue pour enregistrer le processus d'immersion par l'intermédiaire du capteur d'accélération (9).

2. Lance de mesure et de prélèvement d'échantillons (2, 20) selon la revendication 1,
**caractérisée en ce que** l'unité électronique (6, 60) est conçue pour établir un profil de déroulement du processus d'immersion à partir du processus d'immersion enregistré.

3. Lance de mesure et de prélèvement d'échantillons (2, 20) selon l'une des revendications précédentes,
**caractérisée en ce que** l'unité électronique (6, 60) comporte une mémoire (13), en particulier une mémoire annulaire.

4. Lance de mesure et de prélèvement d'échantillons (2, 20) selon l'une des revendications précédentes,
**caractérisée en ce que** des indicateurs d'état (14) sont disposés sur la lance de mesure et de prélèvement d'échantillons (2, 20) et/ou sur le boîtier.

5. Lance de mesure et de prélèvement d'échantillons (2, 20) selon la revendication 4,
**caractérisée en ce que** les indicateurs d'état (14) sont visuels, et l'état dans lequel se trouve la lance de mesure et de prélèvement d'échantillons (2, 20) est affichable par un code couleur.

6. Lance de mesure et de prélèvement d'échantillons (2, 20) selon l'une des revendications précédentes,
**caractérisée en ce que**, après une période préalablement fixée après le retrait de la sonde à immersion (1, 10), l'unité électronique (6, 60) peut être amenée dans un mode économiseur d'énergie et le mode économiseur d'énergie peut être levé lors de la constatation de l'utilisation de la lance de mesure et de prélèvement d'échantillons (2, 20) par l'intermédiaire de l'unité électronique (6, 60).

7. Lance de mesure et de prélèvement d'échantillons (2, 20) selon l'une des revendications précédentes,
**caractérisée en ce que** des moyens auxiliaires pour l'identification claire et sans fil de la lance de mesure et de prélèvement d'échantillons (2, 20) sont appliqués sur la lance de mesure et de prélèvement d'échantillons (2, 20) et/ou **en ce qu'**une localisation peut être effectuée par elle-même par l'intermédiaire de la lance de mesure et de prélèvement d'échantillons (2, 20).

8. Lance de mesure et de prélèvement d'échantillons (20) selon l'une des revendications précédentes,
**caractérisée en ce que** l'unité électronique (60) comporte en tant que premier circuit un transformateur pour la numérisation du signal de capteur et une unité d'analyse (70) et comporte un circuit supplémentaire (21) pour la mise en oeuvre d'un autodiagnostic de la lance de mesure et de prélèvement d'échantillons (20), moyennant quoi le circuit supplémentaire (21) peut être connecté et déconnecté par l'intermédiaire d'un commutateur (19) dans le premier circuit, et le circuit supplémentaire (21) comporte un générateur de tension, en particulier un convertisseur numérique-analogique (90).

9. Lance de mesure et de prélèvement d'échantillons (20) selon la revendication 8,
**caractérisée en ce que** la lance de mesure et de prélèvement d'échantillons (2, 20) comprend un adaptateur à court-circuit (29) dans lequel la sonde à immersion (10) est enfichable, et l'unité d'analyse (70) est conçue, lors de la connexion du circuit supplémentaire (21), pour acheminer dans le générateur de tension au moins une valeur théorique de tension prédéfinie que le générateur de tension introduit dans le circuit supplémentaire, et dans laquelle la valeur théorique de tension peut être guidée par l'intermédiaire d'une liaison électrique par l'intermédiaire de la pièce de contact (3, 30) vers l'adaptateur à court-circuit (29) et ensuite peut être ramenée sous forme de valeur de mesure de tension par l'intermédiaire d'une liaison électrique vers le transformateur pour la numérisation et peut y être numérisée, dans laquelle la valeur de mesure de tension numérisée peut être acheminée dans l'unité d'analyse (70).

10. Lance de mesure et de prélèvement d'échantillons (20) selon l'une des revendications précédentes 8 ou 9,
**caractérisée en ce que** plusieurs valeurs théoriques de tension (18) différentes peuvent être acheminées dans le générateur de tension lors de la connexion du circuit supplémentaire (21) par l'intermédiaire de l'unité d'analyse (70).

11. Lance de mesure et de prélèvement d'échantillons (20) selon la revendication 10,
**caractérisée en ce que** la sonde à immersion (10) comprend plusieurs capteurs de sonde à immersion qui peuvent être diagnostiqués par l'intermédiaire des plusieurs valeurs théoriques de tension (18) différentes.

12. Lance de mesure et de prélèvement d'échantillons (20) selon l'une des revendications précédentes 8 à 11,
**caractérisée en ce que**, par l'intermédiaire de l'unité d'analyse (70), le circuit supplémentaire (21) peut être connecté et déconnecté de manière automatisée dans le premier circuit par l'intermédiaire du commutateur (19).

13. Lance de mesure et de prélèvement d'échantillons (20) selon l'une des revendications précédentes 8 à 12,
**caractérisée en ce qu'**un étalonnage peut être effectué à l'aide de la comparaison de la valeur théorique de tension (18) prédéfinie par l'unité d'analyse (70) par l'intermédiaire du convertisseur numérique/analogique (90) dans le circuit supplémentaire (21) et d'une valeur de mesure de tension (120) mesurée dans le premier circuit.

14. Unité modulaire constituée à partir d'une lance de mesure et de prélèvement d'échantillons (2, 20) selon l'une des revendications précédentes et d'un dispositif d'entreposage (25) pour l'entreposage d'une telle lance de mesure et de prélèvement d'échantillons (2, 20), dans laquelle le boîtier électronique (6, 60) comporte au moins un commutateur magnétique et le dispositif d'entreposage comporte au moins un aimant par l'intermédiaire duquel le commutateur magnétique est activable lors de l'entreposage de la lance de mesure et de prélèvement d'échantillons (2, 20) .

15. Unité modulaire selon la revendication 14,
**caractérisée en ce que** l'unité modulaire comprend un adaptateur à court-circuit (29) dans lequel la sonde à immersion (1, 10) est enfichable.

16. Unité modulaire selon la revendication 14,
**caractérisée en ce que** l'unité modulaire comprend un adaptateur de charge avec lequel l'unité électronique (6, 60) de la lance de mesure et de prélèvement d'échantillons (2, 20) peut être rechargée.

17. Unité modulaire selon l'une des revendications précédentes 14 à 16,
**caractérisée en ce qu'**une mise en oeuvre automatisée d'un autodiagnostic de la lance de mesure et de prélèvement d'échantillons (20) peut être effectuée après l'activation du commutateur magnétique lors de l'entreposage de la lance de mesure et de prélèvement d'échantillons (20).

18. Procédé pour la mesure de grandeurs caractéristiques métallurgiques et pour la prise d'un échantillon de matière fondue liquide avec une lance de mesure et de prélèvement d'échantillons (2, 20) munie d'une pièce de contact (3, 30) et d'une sonde à immersion (1, 10) pouvant être fixée de manière amovible sur la pièce de contact (3, 30), laquelle comprend au moins un capteur pour l'émission d'un signal de capteur caractéristique de la matière fondue,
dans lequel la sonde à immersion (1, 10) est immergée au moins en partie dans la matière fondue liquide, dans lequel le capteur émet le signal caractéristique de la matière fondue,
**caractérisé en ce que** la lance de mesure et de prélèvement d'échantillons (2, 20) comprend un boîtier muni d'une unité électronique (6, 60) dans laquelle est disposée une unité de traitement analogique par l'intermédiaire de laquelle le signal de capteur est au moins prétraité analogiquement, dans lequel le capteur est relié par l'intermédiaire de la pièce de contact (3, 30) au boîtier par l'intermédiaire d'une liaison électrique de telle sorte que le signal de capteur soit guidé par l'intermédiaire de la liaison électrique jusqu'à l'unité électronique (6, 60), et
le processus d'immersion est enregistré par l'intermédiaire d'un capteur d'accélération (9) dans l'unité électronique (6, 60).

19. Procédé pour la mesure selon la revendication 18,
**caractérisé en ce qu'**un profil de déroulement du processus d'immersion est établi par l'intermédiaire du processus d'immersion enregistré par l'intermédiaire de l'unité électronique (6, 60) et **en ce que** le processus d'immersion est considéré comme valide ou non valide à l'aide de ce profil de déroulement.

20. Procédé pour la mesure selon l'une des revendications précédentes 18 à 19,
**caractérisé en ce que**, par l'intermédiaire d'indicateurs d'état (14) disposés sur la lance de mesure et de prélèvement d'échantillons (2, 20) et/ou sur le boîtier, l'état dans lequel se trouve la lance de mesure et de prélèvement d'échantillons (2, 20) est affiché par un code couleur.

21. Procédé pour la mesure selon l'une des revendications précédentes 18 à 20,
**caractérisé en ce que** l'unité électronique (60) comporte dans un premier circuit un transformateur pour la numérisation du signal de capteur et une unité d'analyse (70) et **en ce qu'**un autodiagnostic de la lance de mesure et de prélèvement d'échantillons (20) est mis en oeuvre avec un circuit supplémentaire (21) comportant un générateur de tension, en particulier un convertisseur numérique-analogique (90), **en ce que** le circuit supplémentaire (21) est connecté et déconnecté par l'intermédiaire du commutateur (19) dans le premier circuit.

22. Procédé pour la mesure selon la revendication 21,
**caractérisé en ce que** la sonde à immersion (10) est enfichable dans un adaptateur à court-circuit (29) et l'unité d'analyse (70), lors de la connexion du circuit supplémentaire (21), achemine dans le générateur de tension au moins une valeur théorique de tension (18) prédéfinie que le générateur de tension introduit dans le circuit supplémentaire (21), et dans lequel la valeur théorique de tension (18) est acheminée par l'intermédiaire d'une liaison électrique par l'intermédiaire de la pièce de contact (30) vers l'adaptateur à court-circuit (29) et ensuite est ramenée sous forme de valeur de mesure de tension par l'intermédiaire d'une liaison électrique vers le transformateur et y est numérisée, dans lequel la valeur de mesure de tension (120) numérisée est acheminée dans l'unité d'analyse (70).

23. Procédé pour la mesure selon la revendication 21 ou la revendication 22,
**caractérisé en ce que** plusieurs valeurs théoriques de tension (18) différentes sont acheminées dans le générateur de tension lors de la connexion du circuit supplémentaire (21) par l'intermédiaire de l'unité d'analyse (70).

24. Procédé pour la mesure selon l'une des revendications 21 à 23,
**caractérisé en ce que**, par l'intermédiaire de l'unité d'analyse (70), le circuit supplémentaire (21) est connecté et déconnecté de manière automatisée dans le premier circuit par l'intermédiaire du commutateur (19).

25. Procédé pour la mesure selon l'une des revendications précédentes 21 à 24,
**caractérisé en ce qu'**un étalonnage est effectué à l'aide de la comparaison de la valeur théorique de tension (18) prédéfinie par l'unité d'analyse (70) par l'intermédiaire du générateur de tension dans le circuit supplémentaire et d'une valeur de mesure de tension (120) mesurée dans le premier circuit et numérisée.
